# EUROPÄISCHE PATENTANMELDUNG

(11) **EP 1 712 185 A1**
(43) Veröffentlichungstag der Anmeldung: **18.10.2006**
(21) Anmeldenummer: 05008186.8
(22) Anmeldetag: 14.04.2005
(51) Int. Cl.: A61B 17/02

(54) **Vorrichtung zur Aufrichtung eines eingebrochenen Wirbelkörpers**

(71) Anmelder: Zimmer GmbH, 8404 Winterthur (CH)
(72) Erfinder: Lamartina, Claudio, 20126 Milano (IT); Weidmann, Dirk, 8200 Schaffhausen (CH); Heller, Mathias, 8352 Räterschen (CH)
(74) Vertreter: Manitz, Finsterwald & Partner GbR

(57) **Zusammenfassung**

Vorrichtung zur Aufrichtung eines eingebrochenen Wirbelkörpers, mit einem in seiner wirksamen Länge verstellbaren Distanzhalter (11), der ein Basiselement (13) und ein relativ zu dem Basiselement (13) verstellbares Verstellelement (15) aufweist, wobei der Distanzhalter (11) zwischen Kanülen (63,67), die in den unmittelbar benachbarten Wirbelkörpern gesetzt sind, auf der Haut positionierbar ist und Distanzhaltemittel (17,19) aufweist, von denen wenigstens eines mit dem Basiselement (13) und wenigstens eines mit dem Verstellelement (15) verbunden ist und die zur Fixierung des gegenseitigen Abstands der Kanülen (63,67) an den Eintrittstellen in die Haut ausgelegt sind.

## Beschreibung

Die Erfindung betrifft eine Vorrichtung zur Aufrichtung eines eingebrochenen Wirbelkörpers.

Bei einem Wirbelkörperbruch handelt es sich um eine Fraktur eines Wirbelkörpers der Wirbelsäule. Eine derartige Fraktur kann beispielsweise die Folge eines Unfalls (traumatischer Wirbelkörperbruch) oder osteoporosebedingt sein. Aus dem Stand der Technik sind mehrere Verfahren bekannt, um einen eingebrochenen Wirbelkörper wieder aufzurichten.

Bei der Vertebroplastie wird dem Patienten, der für den Eingriff auf dem Bauch liegt, unter Röntgenkontrolle von hinten perkutan transpedikulär eine Kanüle in den betroffenen Wirbelkörper eingeführt. Zur Aufrichtung und Stabilisierung des Wirbelkörpers wird dann über die Kanüle Knochenzement eingespritzt. Bei diesem Verfahren wird der eingebrochene Wirbelkörper jedoch üblicherweise nicht vollständig aufgerichtet. Die Vertebroplastie hat weiterhin den Nachteil, dass ein größerer Druck auf den Knochenzement notwendig ist, und dass folglich der eingespritzte Knochenzement über die Bruchflächen oder das venöse Blutgefäßsystem wieder austreten kann.

Bei der Kyphoplastie wird dem für den Eingriff auf dem Bauch liegenden Patienten unter Röntgenkontrolle von hinten perkutan transpedikulär über eine Kanüle ein Ballonkatheter in den betroffenen Wirbelkörper eingeführt. Zur Aufrichtung des Wirbelkörpers wird der Ballonkatheter mit einer Röntgenkontrastflüssigkeit aufgeblasen. Nach Ablassen der Kontrastflüssigkeit wird der Ballonkatheter entfernt. In dem von dem Ballonkatheter hinterlassenen Hohlraum wird dann über die Kanüle Knochenzement eingespritzt, um den Wirbelkörper zu stabilisieren. Bei diesem Verfahren wird der eingebrochene Wirbelkörper, auf den die beiden benachbarten Wirbelkörper drücken, jedoch üblicherweise ebenfalls nicht vollständig aufgerichtet. Die Kyphoplastie hat weiterhin den Nachteil, dass die hierfür benötigten Ballonkatheter sehr teuer sind.

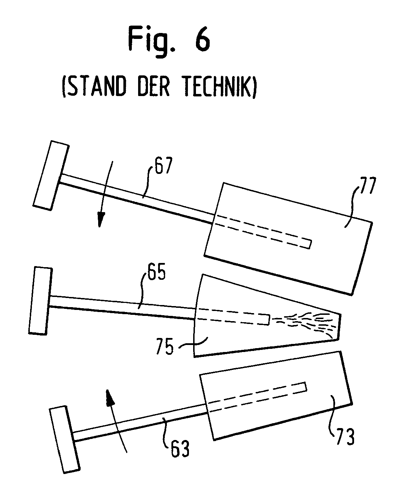

Neben der Vertebroplastie und der Kyphoplastie kommt darum neuerdings des Öfteren die so genannte Lordoplastie zur Anwendung. Hierbei handelt es sich um eine Methode, die in Fig. 6 gezeigt ist und bei der in dem kranial und dem kaudal benachbarten Wirbelkörper 73, 77 des zu behandelnden Wirbelkörpers 75 Kanülen 63, 67 eingeführt und insbesondere einzementiert werden, die als Hebelarme eingesetzt werden, um vor dem Einspritzen von Knochenzement in den eingebrochenen Wirbelkörper 75 diesen zu entlasten. Hierdurch kann die ursprüngliche Wirbelkörperhöhe und Wirbelsäulenkrümmung zumindest annähernd wiederhergestellt werden. Ein Ballonkatheter ist hierfür nicht nötig.

Die Lordoplastie besitzt jedoch den Nachteil, dass die Kanülen beim Hebeln, d.h. beim Zusammendrücken relativ zueinander vergleichsweise stark verbiegen, so dass oftmals die gewünschte Hebelwirkung zumindest nicht vollständig erreicht werden kann. Beispielsweise können sich die Kanülen bereits gegenseitig berühren, ohne dass die gewünschte Entlastung für den eingebrochenen Wirbelkörper erreicht ist. Darüber hinaus werden bei diesem Vorgang die beteiligten Wirbelkörper posterior aneinander gedrückt und somit dort einem erhöhten Druck ausgesetzt, wodurch Knochenstücke in den Nervenkanal gedrückt werden können. Schließlich werden die Hände des Operateurs, der die Hebelarme bzw. Kanülen betätigt und in Position hält, mit hohen Strahlendosen belastet, da die Operation zumindest teilweise unter Röntgenkontrolle stattfindet.

Der Erfindung liegt die Aufgabe zugrunde, eine Vorrichtung zur Aufrichtung eines eingebrochenen Wirbelkörpers zu schaffen, die zumindest einen der vorstehend genannten Nachteile beseitigt.

Die Lösung dieser Aufgabe erfolgt durch die Merkmale des Anspruchs 1 und insbesondere dadurch, dass die Vorrichtung einen in seiner wirksamen Länge verstellbaren Distanzhalter umfasst, der ein Basiselement und ein relativ zu dem Basiselement verstellbares Verstellelement aufweist, wobei der Distanzhalter zwischen Kanülen, die in den unmittelbar benachbarten Wirbelkörpern gesetzt sind, auf der Haut positionierbar ist und Distanzhaltemitteln aufweist, von denen wenigstens eines mit dem Basiselement und wenigstens eines mit dem Verstellelement verbunden ist und die zur Fixierung des gegenseitigen Abstands der Kanülen ausgelegt sind.

Bei der erfindungsgemäßen Vorrichtung ist ein Distanzhalter vorgesehen, der zwischen wenigstens einer Kanüle, die in dem kranial benachbarten Wirbelkörper gesetzt ist, und wenigstens einer Kanüle, die in dem kaudal benachbarten Wirbelkörper gesetzt ist, auf der Haut eines Patienten positionierbar ist. Der Distanzhalter ist in seiner wirksamen Länge verstellbar. Um die Längenverstellbarkeit zu gewährleisten, weist der Distanzhalter ein Basiselement und ein relativ zu dem Basiselement verstellbares Verstellelement auf. Die wirksame Länge des Distanzhalters ist an den gegenseitigen Abstand der Kanülen insbesondere an den Eintrittsstellen in die Haut anpassbar. Insbesondere fixiert der Distanzhalter einen Minimalabstand zwischen den Kanülen. Die Kanülen können beispielsweise Biopsie-Nadeln sein.

Die Anpassung an den gegenseitigen Abstand der Kanülen erfolgt insbesondere bei nicht betätigten Kanülen, d.h. bevor die kraniale Kanüle und die kaudale Kanüle relativ zueinander zusammengedrückt werden. Durch den Distanzhalter kann der gegenseitige Abstand der Kanülen, insbesondere an der Haut oder in der Nähe der Haut, der bei nicht betätigten Kanülen vorliegt, auch bei betätigten Kanülen, d.h. in einem Zustand, in dem die Kanülen relativ zueinander zusammengedrückt werden, beibehalten werden. Hierdurch kann verhindert werden, dass die beteiligten Wirbelkörper posterior aneinander gedrückt und dort jeweils einem erhöhten Druck ausgesetzt werden.

Zur Fixierung des gegenseitigen Abstands der Kanülen sind Distanzhaltemittel vorgesehen. Wenigstens ein Distanzhaltemittel ist mit dem Basiselement und wenigstens ein Distanzhaltemittel ist mit dem Verstellelement verbunden. Das Distanzhaltemittel des Basiselements kann sowohl der kranialen Kanüle als auch der kaudalen Kanüle zugeordnet werden. Das Distanzhaltemittel des Verstellelements wird dann entsprechend der Kanüle der jeweils anderen Richtung zugeordnet.

Bei einem Verfahren, bei dem die erfindungsgemäße Vorrichtung eingesetzt wird, wird auch in den eingebrochenen Wirbelkörper zumindest eine Kanüle gesetzt, um das Einspritzen von Knochenzement in den eingebrochenen Wirbelkörper zu ermöglichen. Bevorzugt sind in dem eingebrochenen und den unmittelbar benachbarten Wirbelkörpern jeweils zwei Kanülen gesetzt.

Vorteilhafte Ausführungsformen der Erfindung sind auch in den Unteransprüchen, der Beschreibung sowie der Zeichnung angegeben.

Gemäß einer bevorzugten Ausführungsform der Erfindung stehen die Distanzhaltemittel nach links und/oder rechts, insbesondere symmetrisch von einer Mittenebene des Distanzhalters von dem Basiselement und dem Verstellelement ab. Für das Basiselement und das Verstellelement kann insbesondere jeweils ein Distanzhaltemittel vorgesehen sein, das als durchgehender Stab oder dergleichen ausgebildet ist und gleichzeitig nach links und rechts absteht. Für das Basiselement und das Verstellelement können jeweils aber auch zwei oder mehr Distanzhaltemittel vorgesehen sein, von denen wenigstens ein Distanzhaltemittel nach links und wenigstens ein Distanzhaltemittel nach rechts absteht. Distanzhaltemittel, die nach links und rechts abstehen, eignen sich besonders gut in den Fällen, in denen jeweils zwei Kanülen für jeden benachbarten Wirbelkörper gesetzt sind.

Gemäß einer weiteren bevorzugten Ausführungsform der Erfindung ist zumindest ein Teil des Basiselements nach Art eines Rahmens ausgebildet, der zwei Seitenteile aufweist. Durch die zumindest teilweise Ausführung des Basiselements als Rahmen kann Material und Gewicht eingespart werden. Darüber hinaus kann Platz für das Verstellelement und/oder den Verstellmechanismus geschaffen werden, um einen besonders kompakten Aufbau der erfindungsgemäßen Vorrichtung zu ermöglichen.

Insbesondere sind in den Seitenteilen des Rahmens Langlöcher ausgebildet, in denen das Verstellelement verstellbar gelagert ist. Durch die Langlöcher in den Seitenteilen des Rahmens werden die Lagerung des Verstellelements an dem Basiselement und gleichzeitig die Längenverstellbarkeit des Verstellelements relativ zu dem Basiselement, insbesondere auf besonders einfache Weise, gewährleistet. Durch die Langlöcher kann zusätzlich Material und Gewicht eingespart werden.

Zur Einstellung verschiedener Relativpositionen zwischen dem Verstellelement und dem Basiselement weist zumindest eines der Langlöcher mehrere Arretierpositionen auf. Hierdurch kann der auf den Abstand der Kanülen insbesondere an den Eintrittsstellen in die Haut in seiner wirksamen Länge angepasste Distanzhalter in der jeweiligen Länge arretiert werden. Die Arretierpositionen sind bevorzugt in Form von kreisbogenförmigen Vertiefungen in den Längsseiten des Langlochs ausgebildet. In einer derartigen Vertiefung kann ein im Querschnitt zumindest teilweise kreisrund ausgeführtes Element des Verstellelements ortsfest und unverrückbar gelagert werden.

Des Weiteren wird erfindungsgemäß vorgeschlagen, dass das Verstellelement ein Verstellteil und ein mit dem Verstellteil verbundenes Bedienteil umfasst, das zwischen einer Freigabestellung und einer Blockierstellung relativ zum Verstellteil umschaltbar ist, wobei bevorzugt das Verstellteil und das Bedienteil gelenkig miteinander verbunden sind. In der Freigabestellung des Bedienelements kann der Distanzhalter in seiner wirksamen Länge entlang der Verstelleinrichtung verstellt und anschließend durch Überführen in die Blockierstellung mit der eingestellten Länge arretiert werden. Bei einer gelenkigen Verbindung zwischen Verstellteil und Bedienteil wird ermöglicht, dass beim Umschalten des Bedienteils das Verstellteil unbewegt in seiner Position verharren kann.

Bevorzugt ragt das Bedienteil durch einen in einem Rahmen des Basiselements ausgebildeten Rahmenschlitz hindurch. Dadurch wird das Bedienteil von dem Rahmen gewissermaßen eingefasst, so dass ein besonders kompakter Aufbau ermöglicht wird.

Bevorzugt ist das insbesondere bogenförmige Bedienteil drehstarr mit einer am Basiselement gelagerten Achse des Verstellelements verbunden. Die Umschaltbewegung des Bedienteils zwischen einer Freigabestellung und einer Blockierstellung kann somit eine Drehbewegung sein. Da die Achse drehstarr mit dem Bedienteil verbunden ist, wird bei einer Umschaltbewegung des Bedienteils sowohl die am Basiselement gelagerte Achse als auch das Bedienteil gedreht.

Weiterhin bevorzugt ist an dem Verstellteil zumindest ein Distanzhaltemittel ausgebildet, wobei das Verstellteil gelenkig mit einer am Basiselement gelagerten Achse des Verstellelements, die insbesondere der drehstarr mit dem Bedienteil verbundenen Achse entspricht, verbunden ist. Bei einer Drehbewegung des Bedienteils und somit der Achse kann das Verstellteil unbewegt in seiner Position verharren, da das Verstellteil gelenkig mit der Achse verbunden ist.

Besonders vorteilhaft ist es, wenn die Achse in Langlöchern verstellbar gelagert ist, insbesondere in Langlöchern von Seitenteilen eines Rahmens des Basiselements. Die Achse ist dabei entlang der Langlöcher verstellbar. Zusätzlich ermöglicht die Achse, dass wenigstens ein Teil des Verstellelements, wie insbesondere das Bedienteil, relativ zu dem Basiselement drehbar ist. Insbesondere ist die Achse an zwei gegenüberliegenden Seiten abgeplattet, wobei bevorzugt der Durchmesser der Achse im Wesentlichen dem Durchmesser von kreisbogenförmigen Vertiefungen in den Längsseiten eines Langlochs entspricht. Insbesondere kann durch Drehung die abgeplattete Achse und damit das Verstellelement ortsfest an dem Basiselement arretiert und/oder die Arretierung gelöst werden.

Das Bedienteil kann wenigstens einen Abstützschenkel umfassen, der außerhalb eines Rahmens des Basiselements angeordnet ist. Der Abstützschenkel kann als Auflagepunkt oder Auflagebereich für ein Griffteil vorgesehen sein, das auf den Distanzhalter aufsetzbar ist und an anderer Stelle noch näher erläutert wird.

Das Verstellteil ist bevorzugt entlang einer Aussparung des Basiselements verstellbar, die an dessen Unterseite verläuft. Dies ist insbesondere deshalb von Vorteil, da das Verstellelement mit einem Distanzhaltemittel versehen ist, das hierdurch besonders nahe an der Haut positionierbar ist und den Abstand der Kanüle unmittelbar an der Eintrittsstelle in die Haut fixieren kann. Insbesondere kann hierdurch das Verstellteil einen Teil der Auflagefläche des Distanzhalters auf der Haut bilden.

Gemäß einer vorteilhaften Ausführungsform der Erfindung ist für die kraniale und kaudale Kanüle jeweils ein insbesondere schaufelartiges Griffteil vorgesehen, das auf dem Distanzhalter derart aufsetzbar ist, dass das Griffteil eine Anlagefläche für die jeweilige Kanüle bereitstellt. Durch Zusammendrücken der Griffteile relativ zueinander werden somit auch die aus dem Körper herausragenden Bereiche der Kanülen relativ zueinander zusammengedrückt. Da die Kanülen über einen verhältnismäßig großen Teilbereich ihrer Länge an den Griffteilen anliegen können, kann ein übermäßiges Verbiegen der aus dem Körper herausragenden Bereiche der Kanülen wirksam verhindert werden.

Um die Anlageflächen bereitzustellen, können die Griffteile jeweils einen plattenartigen Basisabschnitt aufweisen, der insbesondere zum Aufsetzen auf dem Distanzhalter mittig geschlitzt und/oder zur Führung der Kanüle mit seitlichen Anlagebegrenzungen versehen ist. Der mittige Schlitz erlaubt, dass sich der Basisabschnitt in aufgesetztem Zustand des Griffteils auch links und rechts des Distanzhalters erstrecken kann, so dass der Kanüle über einen möglichst großen Teilbereich hinweg eine Anlagefläche zur Verfügung steht. Die seitlichen Anlagebegrenzungen verhindern, dass die Kanüle beim Hebeln nach links oder rechts ausweicht, um dem Druck des Griffteils bzw. des Basisabschnitts zu entgehen.

Des Weiteren können die Griffteile jeweils einen insbesondere S-förmig ausgebildeten Betätigungsabschnitt aufweisen, wobei in aufgesetztem Zustand die Betätigungsabschnitte einen größeren Abstand voneinander aufweisen als Basisabschnitte der Griffteile. Die Griffteile werden bevorzugt über die Betätigungsabschnitte betätigt. Um einen ausreichenden Hebelweg und/oder Platz für Handgriffe der Kanülen bereitzustellen, kann der Abstand der Betätigungsabschnitte größer als der Abstand der Basisabschnitte sein.

Nach einer besonderen Ausgestaltung der Erfindung sind die Griffteile über eine einstellbare Fixiereinrichtung, insbesondere eine Stellschraube und/oder eine Mutter, miteinander verbindbar. Durch die Fixiereinrichtung kann in aufgesetztem Zustand der Abstand zwischen den Griffteilen fixiert werden. Dies gilt insbesondere auch für einen aufgesetzten Zustand, in dem die beiden Griffteile bereits relativ zueinander zusammengedrückt sind. Der Operateur ist somit nicht gezwungen, während des Einspritzens von Knochenzement in den eingebrochenen Wirbelkörper die betätigten Griffteile bzw. Kanülen per Hand in Position zu halten, so dass die Strahlenbelastung für den Operateur minimiert werden kann. Es ist auch möglich, dass die Fixiereinrichtung nicht nur zur Fixierung der beiden Griffteile, sondern zusätzlich zum Zusammendrücken der beiden Griffteile relativ zueinander an sich verwendet wird.

Um eine besonders einfache Montage der Fixiereinrichtung zu ermöglichen, weist zumindest ein Griffteil einen Einführschlitz auf, durch den die Fixiereinrichtung in das Griffteil einführbar ist. Hierdurch kann die Stellschraube, insbesondere gemeinsam mit einer Mutter, relativ zu dem Griffteil gewissermaßen in dieses eingeschwenkt werden. Der Einführschlitz kann teilweise in einem Basisabschnitt und teilweise in einem Betätigungsabschnitt des Griffteils ausgebildet sein, wobei bevorzugt der im Betätigungsabschnitt ausgebildete Teil des Einführschlitzes die Durchführung der Mutter erlaubt.

Ferner kann erfindungsgemäß vorgesehen sein, dass das Basiselement Abstützmittel aufweist, die insbesondere auf gleicher Höhe wie Abstützmittel des Verstellelements angeordnet sind. Die Abstützmittel sind zur Abstützung der auf dem Distanzhalter aufgesetzten Griffteile vorgesehen, insbesondere wenn eine Fixiereinrichtung zur Fixierung des Abstands zwischen den Griffteilen noch nicht montiert ist.

Bei einem Verfahren zur Aufrichtung eines eingebrochenen Wirbelkörpers wird jeweils wenigstens eine Kanüle in den eingebrochenen und die unmittelbar benachbarten Wirbelkörper gesetzt, wird zwischen der kranialen Kanüle und der kaudalen Kanüle ein in seiner wirksamen Länge verstellbarer Distanzhalter derart auf der Haut positioniert, dass der gegenseitige Abstand der Kanülen insbesondere an den Eintrittsstellen in die Haut fixiert ist, werden die kraniale Kanüle und die kaudale Kanüle der unmittelbar benachbarten Wirbelkörper an aus dem Körper herausragenden Bereichen relativ zueinander zusammengedrückt, um den eingebrochenen Wirbelkörper zu entlasten, und wird durch die entsprechende Kanüle Knochenzement in den eingebrochenen Wirbelkörper eingespritzt.

Bevorzugt wird nach der Positionierung des Distanzhalters und vor dem Zusammendrücken der Kanülen ein Bedienteil eines Verstellelements des Distanzhalters aus einer Freigabestellung in eine Blockierstellung relativ zum Verstellteil umgeschaltet, wobei bevorzugt der Distanzhalter zunächst in der Freigabestellung in seiner wirksamen Länge entlang der Verstellrichtung verstellt und dann durch Umschalten des Bedienteils in die Blockierstellung in der verstellten wirksamen Länge, die dem Abstand zwischen der kranialen Kanüle und der kaudalen Kanüle entspricht, arretiert wird.

Des Weiteren kann nach der Positionierung des Distanzhalters, insbesondere nach dem Verstellen der wirksamen Länge des Distanzhalters, und vor dem Zusammendrücken der Kanülen kranial und kaudal jeweils ein insbesondere schaufelartiges Griffteil zwischen die Kanüle und ein Distanzhaltemittel des Distanzhalters eingeführt und derart auf den Distanzhalter aufgesetzt werden, dass das Griffteil eine Anlagefläche für die jeweilige Kanüle bereitstellt.

Vorzugsweise werden die Griffteile relativ zueinander zusammengedrückt, um die aus dem Körper herausragenden Bereiche der Kanülen relativ zueinander zusammenzudrücken.

Nach dem Zusammendrücken der Kanülen kann der Abstand zwischen den Griffteilen mittels einer einstellbaren Fixiereinrichtung fixiert werden.

Die Erfindung wird nachfolgend beispielhaft anhand der Zeichnung beschrieben. In dieser zeigen:
- Fig. 1: eine perspektivische Ansicht eines Distanzhalters einer erfindungsgemäßen Vorrichtung,
- Fig. 2: perspektivische Ansichten von Bestandteilen des in Fig. 1 gezeigten Distanzhalters,
- Fig. 3: perspektivische Ansichten von Griffteilen einer erfindungs-gemäßen Vorrichtung,
- Fig. 4: verschiedene Ansichten einer erfindungsgemäßen Vorrichtung mit dem Distanzhalter von Fig. 1 und den Griffteilen von Fig. 3,
- Fig. 5: eine weitere Ausführungsform einer erfindungsgemäßen Vorrichtung, wobei zusätzlich Kanülen gezeigt sind, die in dem eingebrochenen und den unmittelbar benachbarten Wirbelkörpern gesetzt sind, und
- Fig. 6: eine Darstellung eines aus dem Stand der Technik bekannten Operationsverfahrens.

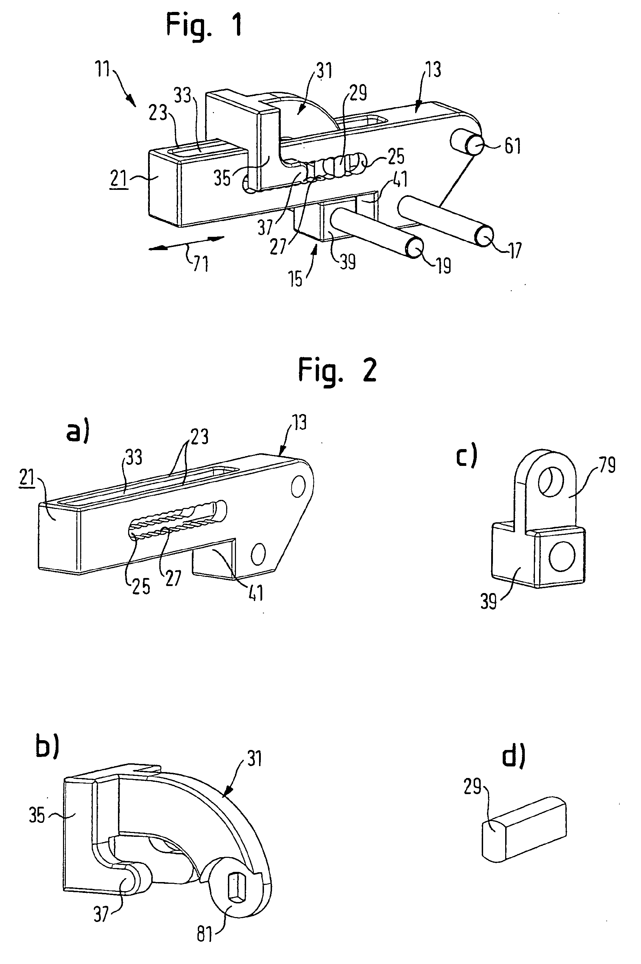

Fig. 1 zeigt einen Distanzhalter 11 einer erfindungsgemäßen Vorrichtung zur Aufrichtung eines eingebrochenen Wirbelkörpers 75, der lediglich im Zusammenhang mit einem aus dem Stand der Technik bekannten Operationsverfahren in Fig. 6 gezeigt ist. Der Distanzhalter umfasst ein Basiselement 13, das zusätzlich in Fig. 2a dargestellt ist, und ein mehrteiliges Verstellelement 15, dessen Einzelteile 29, 31, 39 zusätzlich in den Fig. 2c bis 2d dargestellt sind. Das Verstellelement 15 ist entlang einer Verstellrichtung 71 an dem Basiselement 13 verstellbar, um die wirksame Länge zwischen einer stabförmigen Distanzhaltewelle 17, die an einem Kopfabschnitt des Basiselements 13 angebracht ist, und einer ebenfalls stabförmigen Distanzhaltewelle 19, die an dem Verstellelement 15 angebracht ist, einzustellen.

Die Distanzhaltewelle 17 und die Distanzhaltewelle 19 erstrecken sich jeweils senkrecht zu der Verstellrichtung 71 des Verstellelements 15 bzw. zu der Längsachse des Distanzhalters 11, sind jeweils durch eine in dem Basiselement 13 bzw. in dem Verstellelement 15 ausgebildete zylinderförmige Bohrung hindurchgeführt und stehen zu beiden Seiten, insbesondere symmetrisch zu einer Mittenebene des Distanzhalters 11 von dem Basiselement 13 bzw. dem Verstellelement 15 ab.

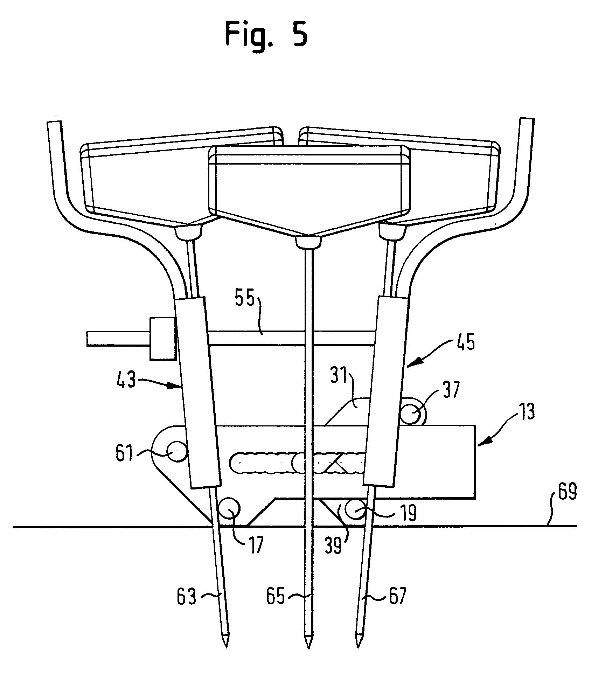

Der Distanzhalter 11 ist zwischen Kanülen 63, 67, die lediglich im Zusammenhang mit einer weiteren Ausführungsform einer erfindungsgemäßen Vorrichtung in Fig. 5 sowie in Fig. 6 gezeigt sind, auf der Haut 69 (Fig. 5) eines Patienten, der während der Operation auf dem Bauch liegt, positionierbar. Die Kanülen 63 und 67 sind in den unmittelbar benachbarten Wirbelkörpern 73, 77 (Fig. 6) des eingebrochenen Wirbelkörpers 75 gesetzt. In den Fig. 5 und 6 sind in den benachbarten Wirbelkörpern 73, 77 sowie in dem eingebrochenen Wirbelkörper 75 jeweils zwei Kanülen 63, 65, 67 links und rechts der Symmetrieebene der Wirbelkörper 73, 75, 77 gesetzt. Die wirksame Länge des Distanzhalters 11 wird derart eingestellt, dass die Distanzhaltewellen 17, 19 innen an den Kanülen 63, 67 anliegen, um den gegenseitigen Abstand der Kanülen 63, 67 an den Eintrittsstellen in die Haut 69 zu fixieren.

Das Basiselement 13 umfasst neben dem Kopfabschnitt einen Abschnitt, der nach Art eines Rahmens 21 ausgebildet ist. In den beiden Seitenteilen 23 des Rahmens 21 ist jeweils ein Langloch 25 ausgebildet, in denen eine an zwei gegenüberliegenden Seiten abgeplattete Achse 29 verstellbar gelagert ist. Jedes der beiden Langlöcher 25 weist zur Einstellung verschiedener Relativpositionen zwischen dem Verstellelement 15 und dem Basiselement 13 mehrere Arretierpositionen auf, die in Form von kreisbogenförmigen Vertiefungen 27 in den Längsseiten des Langlochs 25 ausgebildet sind.

Das Verstellelement 15 umfasst ein Verstellteil 39 mit einer zylinderförmigen Bohrung, durch die die Distanzhaltewelle 19 gesteckt ist. Darüber hinaus weist das Verstellteil 39 einen Gelenkabschnitt 79 auf, der ebenfalls eine zylinderförmige Bohrung besitzt und durch die sich in zusammengesetztem Zustand des Verstellelements 15 gemäß Fig. 1 die Achse 29 erstreckt. Das Verstellteil 39 und die Achse 29 sind gelenkig miteinander verbunden, d.h. die Achse 29 ist innerhalb der Zylinderbohrung des Gelenkabschnitts 79 des Verstellteils 39 frei drehbar. Das Verstellteil 39, insbesondere der Abschnitt 79, an dem die Distanzhaltewelle 19 ausgebildet ist, ist entlang einer Aussparung 41 an der Unterseite des Basiselements 13 verstellbar. Der Distanzhalter 11 liegt somit über den festen Kopfabschnitt und das verstellbare Verstellteil 39 auf der Haut auf.

Das Verstellelement 15 umfasst weiterhin ein Bedienteil 31. Das Bedienteil 31 ist bogenförmig ausgebildet und ragt durch einen in dem Rahmen 21 des Basiselements 13 ausgebildeten Rahmenschlitz 33 hindurch. Das Bedienteil 31 umfasst ebenfalls einen Gelenkabschnitt 81, in dem eine Bohrung für die Achse 29 ausgebildet ist und der in zusammengesetztem Zustand des Verstellelements 15 gemäß Fig. 1 neben dem Gelenkabschnitt 79 des Verstellteils 39 und an diesem anliegend angeordnet ist. Die Kontur der Bohrung des Gelenkabschnitts 81 des Basiselements 13 ist der Kontur der abgeplatteten Achse 29 angepasst, so dass das Bedienteil 31 drehstarr mit der Achse 29 verbunden ist.

Das Bedienteil 31 ist zwischen einer Blockierstellung, in der das Verstellelement 15 unverstellbar an dem Basiselement 13 arretiert ist und die in Fig. 1 gezeigt ist, und einer Freigabestellung, in der das Verstellelement 15 entlang der Verstellrichtung 71 relativ zu dem Basiselement 13 verstellbar ist, umschaltbar. In der Arretierstellung greifen die nicht abgeplatteten Seiten der Achse 29 in die kreisbogenförmigen Vertiefungen 27 in den Längsseiten der Langlöcher 25 form- und kraftschlüssig sein, so dass eine Verstellbarkeit entlang der Verstellrichtung 71 wirksam unterbunden wird. Dies wird insbesondere dadurch ermöglicht, dass der Durchmesser der Achse 29 im Wesentlichen dem Durchmesser der kreisbogenförmigen Vertiefungen 27 in den Längsseiten der Langlöcher 25 entspricht.

In der Freigabestellung, die dadurch erreicht wird, dass das Bedienteil 31 um die Achse 29 geschwenkt wird, wobei aufgrund der drehstarren Verbindung zwischen dem Bedienteil 31 und der Achse 29 auch die Achse 29 mitdreht. Dies führt dazu, dass die nicht abgeplatteten Seiten der Achse 29 und die Vertiefungen 27 in den Längsseiten der Langlöcher 25 außer Eingriff gesetzt werden, so dass das Verstellelement 15 in Verstellrichtung 71 relativ zu dem Basiselement 13 verstellbar werden kann.

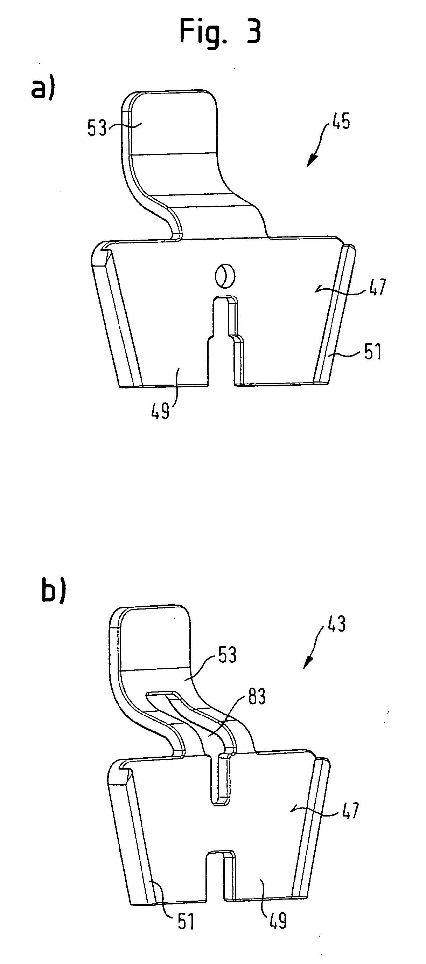

Das Bedienteil 31 weist an seinem Ende, das nach oben aus dem Rahmenschlitz 33 des Basiselements 13 herausragt, sowohl links als auch rechts jeweils einen Abstützschenkel 35 auf, der sich außerhalb des Rahmens 21 des Basiselements 13 seitlich der Seitenteile 23 des Rahmens 21 erstreckt. An den Abstützschenkeln 35 ist jeweils ein Vorsprung 37 ausgebildet, der in Verstellrichtung 71 gerichtet ist und in Analogie zu einer Abstützwelle 61, die über eine Zylinderbohrung an dem Kopfabschnitt des Basiselements 13 befestigt ist und symmetrisch nach links und rechts von dem Distanzhalter 11 absteht, als Abstützmittel für Griffteile 43, 45 dient, die in Fig. 3 gezeigt sind.

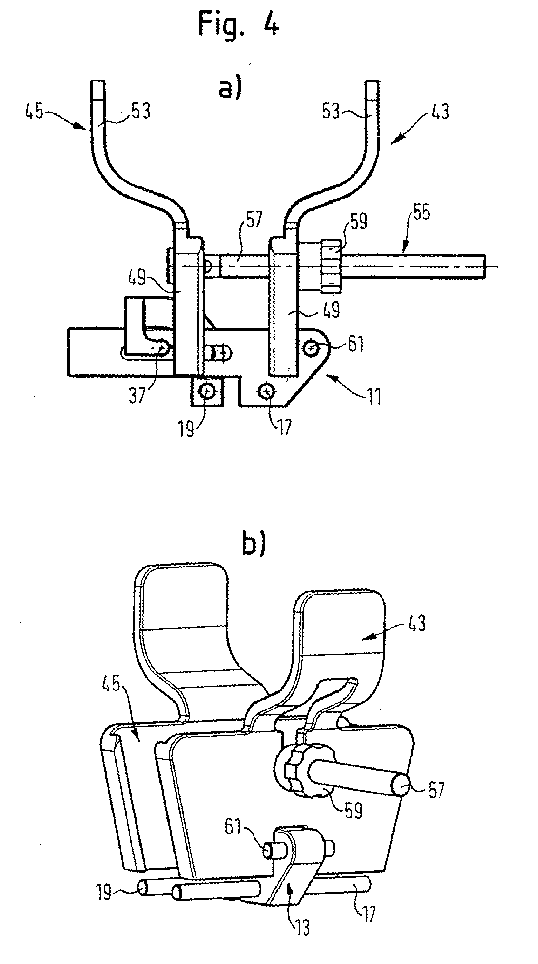

Die Griffteile 43, 45, die jeweils schaufelartig ausgebildet sind, sind kranial und kaudal auf den Distanzhalter 11 derart aufsetzbar, dass die Griffteile 43, 45 eine Anlagefläche 47 für die kranialen und kaudalen Kanülen 63, 67 bereitstellen. Die Griffteile 43, 45 weisen jeweils einen plattenartigen Basisabschnitt 49 auf, der zum Aufsetzen auf den Distanzhalter 11 mittig geschlitzt ist. Darüber hinaus ist der Basisabschnitt 49 mit seitlichen Anlagebegrenzungen 51 versehen, um eine Führung für die Kanülen 63, 67 bereitzustellen, so dass diese bei einer Betätigung der Griffteile 43, 45 nicht nach links oder rechts ausweichen können. Ferner weisen die Griffteile 43, 45 jeweils einen S-förmig ausgebildeten Betätigungsabschnitt 53 auf, die unmittelbar an die Basisabschnitte 49 anschließen. In auf den Distanzhalter 11 aufgesetztem Zustand (Fig. 4) weisen die Betätigungsabschnitte 53 einen größeren Abstand voneinander auf als die Basisabschnitte 49.

Schließlich umfasst die erfindungsgemäße Vorrichtung zur Aufrichtung eines eingebrochenen Wirbelkörpers 75 eine in Fig. 4 gezeigte einstellbare Fixiereinrichtung 55, über die die beiden Griffteile 43, 45 in auf den Distanzhalter 11 aufgesetztem Zustand miteinander verbunden sind. Die Fixiereinrichtung 55 umfasst dabei eine Stellschraube 57, die durch eine in dem Griffteil 45 ausgebildete kreisrunde Öffnung und einen in dem Griffteil 43 ausgebildeten Einführschlitz 83 ein- bzw. hindurchgeführt ist, wobei auf das aus dem Einführschlitz 83 heraustretende Ende der Stellschraube 57 eine Mutter 59 aufgeschraubt ist, um die gegenseitige Stellung der beiden Griffteile 43, 45 zu fixieren.

Eine weitere, zweite Ausführungsform einer erfindungsgemäßen Vorrichtung zur Aufrichtung eines eingebrochenen Wirbelkörpers 75 ist in Fig. 5 gezeigt, wobei jeweils gleiche oder entsprechende Teile mit denselben Bezugszeichen bezeichnet sind, so dass lediglich die Abweichungen der zweiten Ausführungsform gegenüber der in den Fig. 1 bis 4 dargestellten ersten Ausführungsform erläutert werden.

Bei der Vorrichtung gemäß Fig. 5 ist an dem oberen Ende des Bedienteils 31 eine Abstützwelle 37 angebracht, die nach links und rechts von dem Distanzhalter 11 absteht. Die Abstützwelle 37 übernimmt dabei die Funktion der Vorsprünge 37 der ersten Ausführungsform. Letztendlich unterscheidet sich die zweite Ausführungsform von der ersten Ausführungsform lediglich dadurch, dass die dem Basiselement 13 zugeordneten Abstützmittel 61 nicht auf gleicher Höhe wie die dem Verstellelement 15 zugeordneten Abstützmittel 37 angeordnet sind.

Nachfolgend wird ein Verfahren zur Aufrichtung eines eingebrochenen Wirbelkörpers unter Bezugnahme auf die Figuren beschrieben.

Zunächst werden jeweils zwei Kanülen 63, 65, 67 in den eingebrochenen Wirbelkörper 75 und die unmittelbar benachbarten Wirbelkörper 73, 77 gesetzt. Danach wird in die unmittelbar benachbarten Wirbelkörper 73, 77 Knochenzement eingespritzt. Sobald der in die benachbarten Wirbelkörper 73, 77 eingespritzte Knochenzement ausgehärtet ist, kann das Operationsverfahren fortgesetzt werden. Natürlich ist es auch möglich, dass die für den eingebrochenen Wirbelkörper 75 vorgesehenen Kanülen 65 erst nach dem Einspritzen des Knochenzements in die unmittelbar benachbarten Wirbelkörper 73, 77 und/oder dem Aushärten des Knochenzements in den benachbarten Wirbelkörpern 73, 77 gesetzt werden.

Als Nächstes wird der in seiner wirksamen Länge verstellbare erfindungsgemäße Distanzhalter 11 derart auf der Haut 69 eines Patienten positioniert, dass der gegenseitige Abstand der kranialen und kaudalen Kanülen 63, 67 an den Eintrittsstellen in die Haut fixiert ist. Hierfür wird in der Freigabestellung die wirksame Länge des Distanzhalters 11 derart eingestellt, dass die Distanzhaltewelle 17 und die Distanzhaltewelle 19 jeweils an den Innenseiten der kranialen bzw. kaudalen Kanülen 63, 67 anstehen. Ist dies erreicht, wird das Bedienteil 31 von der Freigabestellung in die Blockierstellung umgeschaltet, um die Länge des Distanzhalters 11 in dem entsprechenden Abstand zu arretieren. Natürlich ist es auch möglich, dass das Setzen der Kanülen 65 in den eingebrochenen Wirbelkörper 75, das Einspritzen des Knochenzements in die unmittelbar benachbarten Wirbelkörper 73, 77 und/oder das Aushärten des Knochenzements in den benachbarten Wirbelkörpern 73, 77 während oder nach der Positionierung oder der Längeneinstellung des Distanzhalters 11 durchgeführt wird.

Anschließend werden die beiden Griffteile 43, 45 zwischen die kranialen und kaudalen Kanülen 63, 67 und die Distanzhaltewellen 17, 19 des Distanzhalters 11 eingeführt und derart auf den Distanzhalter 11 aufgesetzt, dass die Griffteile 43, 45 eine Anlagefläche 47 für die Kanülen 63, 67 bereitstellen. In Verstellrichtung 71 - jeweils von außen nach innen-gesehen, liegt somit folgende Reihenfolge vor (Fig. 5): Abstützwelle 61 bzw. 37 - Griffteil 43 bzw. 45 - Kanüle 63 bzw. 67 - Distanzhaltewelle 17 bzw. 19.

Dann werden die Griffteile 43, 45 relativ zueinander zusammengedrückt, so dass die aus dem Körper des Patienten herausragenden Bereiche der Kanülen 63, 67 ebenfalls relativ zueinander zusammengedrückt werden.

Hierdurch wird erreicht, dass der eingebrochene Wirbelkörper 75 entlastet wird. Die beiden Distanzhaltewellen 17 und 19 wirken dabei als Drehpunkt oder "Knickpunkt" für die kranialen und kaudalen Kanülen 63, 67. Die durch die Basisabschnitte 49 der Griffteile 43, 45 beaufschlagten Bereiche der Kanülen 63, 67 werden dabei nicht verbogen.

Sobald der gewünschte Abstand zwischen den Griffteilen 43, 45 erreicht ist, der einer gewünschten Entlastung des eingebrochenen Wirbelkörpers 75 entspricht, wird der Abstand zwischen den Griffteilen 43, 45 mittels der Fixiereinrichtung 55 fixiert. Dies wird insbesondere dadurch erreicht, dass die Mutter 59 bis auf Anschlag an das Griffteil 43 nachgezogen wird. Prinzipiell ist es auch möglich, dass die Fixiereinrichtung 55 zum Zusammendrücken der beiden Griffteile 43, 45 relativ zueinander an sich verwendet wird.

Schließlich wird über die Kanülen 65 Knochenzement in den eingebrochenen Wirbelkörper 75 eingespritzt, um den eingebrochenen Wirbelkörper 75 zu stabilisieren. Nach Aushärten des in den eingebrochenen Wirbelkörper 75 eingespritzten Knochenzements können die erfindungsgemäße Vorrichtung und die in den beteiligten Wirbelkörpern 73, 75, 77 gesetzten Kanülen 63, 65, 67 entfernt werden. Das Operationsverfahren findet zumindest teilweise unter Röntgenkontrolle statt.

Die erfindungsgemäße Vorrichtung zur Aufrichtung eines eingebrochenen Wirbelkörpers 75, die zumindest den vorstehend erläuterten Distanzhalter 11 und bevorzugt auch die beiden vorstehend erläuterten Griffteile 43, 45 und die Fixiereinrichtung 55 umfasst, ermöglicht, dass sämtliche im Zusammenhang mit dem Stand der Technik geschilderten Nachteile bislang bekannter Operationstechniken beseitigt werden können. Insbesondere kann durch die erfindungsgemäße Vorrichtung eine bessere Versorgung von eingebrochenen Wirbelkörpern gewährleistet werden.

### Bezugszeichenliste

- 11: Distanzhalter
- 13: Basiselement
- 15: Verstellelement
- 17: Distanzhaltewelle
- 19: Distanzhaltewelle
- 21: Rahmen
- 23: Seitenteil
- 25: Langloch
- 27: Vertiefung
- 29: abgeplattete Achse
- 31: Bedienteil
- 33: Rahmenschlitz
- 35: Abstützschenkel
- 37: Vorsprung/Abstützwelle
- 39: Verstellteil
- 41: Aussparung
- 43: Griffteil
- 45: Griffteil
- 47: Anlagefläche
- 49: Basisabschnitt
- 51: Anlagebegrenzung
- 53: Betätigungsabschnitt
- 55: Fixiereinrichtung
- 57: Stellschraube
- 59: Mutter
- 61: Abstützwelle
- 63: Kanüle
- 65: Kanüle
- 67: Kanüle
- 69: Haut
- 71: Verstellrichtung
- 73: Wirbelkörper
- 75: Wirbelkörper
- 77: Wirbelkörper
- 79: Gelenkabschnitt
- 81: Gelenkabschnitt
- 83: Einführschlitz

## Patentansprüche

1. Vorrichtung zur Aufrichtung eines eingebrochenen Wirbelkörpers (75), mit einem in seiner wirksamen Länge verstellbaren Distanzhalter (11), der ein Basiselement (13) und ein relativ zu dem Basiselement (13) verstellbares Verstellelement (15) aufweist, wobei der Distanzhalter (11) zwischen Kanülen (63, 67), die in den unmittelbar benachbarten Wirbelkörpern (73, 77) gesetzt sind, auf der Haut (69) positionierbar ist und Distanzhaltemittel (17, 19) aufweist, von denen wenigstens eines mit dem Basiselement (13) und wenigstens eines mit dem Verstellelement (15) verbunden ist und die zur Fixierung des gegenseitigen Abstands der Kanülen (63, 67) ausgelegt sind.

2. Vorrichtung nach Anspruch 1,
**dadurch gekennzeichnet,**
**dass** die Distanzhaltemittel (17, 19) nach links und/oder rechts von dem Basiselement (13) und dem Verstellelement (15) abstehen.

3. Vorrichtung nach Anspruch 1 oder 2,
**dadurch gekennzeichnet,**
**dass** zumindest ein Teil (21) des Basiselements (13) nach Art eines Rahmens ausgebildet ist, der zwei Seitenteile (23) aufweist.

4. Vorrichtung nach Anspruch 3,
**dadurch gekennzeichnet,**
**dass** in den Seitenteilen (23) des Rahmens (21) Langlöcher (25) ausgebildet sind, in denen das Verstellelement (15) verstellbar gelagert ist.

5. Vorrichtung nach Anspruch 4,
**dadurch gekennzeichnet,**
**dass** zumindest eines der Langlöcher (25) mehrere Arretierpositionen zur Einstellung verschiedener Relativpositionen zwischen dem Verstellelement (15) und dem Basiselement (13) aufweist, wobei bevorzugt die Arretierpositionen in Form von kreisbogenförmigen Vertiefungen (27) in den Längsseiten des Langlochs (25) ausgebildet sind.

6. Vorrichtung nach einem der vorhergehenden Ansprüche,
**dadurch gekennzeichnet,**
**dass** das Verstellelement (15) ein Verstellteil (39) und ein mit dem Verstellteil (39) verbundenes Bedienteil (31) umfasst, das zwischen einer Freigabestellung und einer Blockierstellung relativ zum Verstellteil (39) umschaltbar ist, wobei bevorzugt das Verstellteil (39) und das Bedienteil (31) gelenkig miteinander verbunden sind.

7. Vorrichtung nach Anspruch 6,
**dadurch gekennzeichnet,**
**dass** das Bedienteil durch einen in einem Rahmen (21) des Basiselements (13) ausgebildeten Rahmenschlitz (33) hindurchragt.

8. Vorrichtung nach Anspruch 6 oder 7,
**dadurch gekennzeichnet,**
**dass** das insbesondere bogenförmige Bedienteil (31) drehstarr mit einer am Basiselement (13) gelagerten Achse (29) des Verstellelements (15) verbunden ist.

9. Vorrichtung nach einem der Ansprüche 6 bis 8,
**dadurch gekennzeichnet,**
**dass** an dem Verstellteil (39) zumindest ein Distanzhaltemittel (19) ausgebildet ist, wobei das Verstellteil (39) gelenkig mit einer am Basiselement (13) gelagerten Achse (29) des Verstellelements (15) verbunden ist.

10. Vorrichtung nach Anspruch 8 oder 9,
**dadurch gekennzeichnet,**
**dass** die insbesondere an zwei gegenüberliegenden Seiten abgeplattete Achse in Langlöchern (25) des Basiselements (13) verstellbar gelagert ist, wobei bevorzugt der Durchmesser der Achse (29) im Wesentlichen dem Durchmesser von kreisbogenförmigen Vertiefungen (27) in den Längsseiten eines Langlochs (25) entspricht.

11. Vorrichtung nach einem der Ansprüche 6 bis 10,
**dadurch gekennzeichnet,**
**dass** das Bedienteil (31) wenigstens einen Abstützschenkel (35) umfasst, der außerhalb eines Rahmens (21) des Basiselements (13) angeordnet ist.

12. Vorrichtung nach einem der vorhergehenden Ansprüche,
**dadurch gekennzeichnet,**
**dass** das Verstellteil (39) entlang einer Aussparung (41) des Basiselements (13) verstellbar ist, die an dessen Unterseite verläuft.

13. Vorrichtung nach einem der vorhergehenden Ansprüche,
**dadurch gekennzeichnet,**
**dass** für die kraniale und kaudale Kanüle (63, 67) jeweils ein insbesondere schaufelartiges Griffteil (43, 45) vorgesehen ist, das auf den Distanzhalter (11) derart aufsetzbar ist, dass das Griffteil (43, 45) eine Anlagefläche (47) für die jeweilige Kanüle (63, 67) bereitstellt.

14. Vorrichtung nach Anspruch 13,
**dadurch gekennzeichnet,**
**dass** die Griffteile (43, 45) jeweils einen plattenartigen Basisabschnitt (49) aufweisen, der insbesondere zum Aufsetzen auf den Distanzhalter (11) mittig geschlitzt und/oder zur Führung der Kanüle (63, 67) mit seitlichen Anlagebegrenzungen (51) versehen ist.

15. Vorrichtung nach Anspruch 13 oder 14,
**dadurch gekennzeichnet,**
**dass** die Griffteile (43, 45) jeweils einen insbesondere S-förmig ausgebildeten Betätigungsabschnitt (53) aufweisen, wobei im aufgesetzten Zustand die Betätigungsabschnitte (53) einen größeren Abstand voneinander aufweisen als Basisabschnitte (49) der Griffteile (43, 45).

16. Vorrichtung nach einem der Ansprüche 13 bis 15,
**dadurch gekennzeichnet,**
**dass** die Griffteile (43, 45) über eine einstellbare Fixiereinrichtung (55), insbesondere eine Stellschraube (57) und/oder eine Mutter (59), miteinander verbindbar sind.

17. Vorrichtung nach Anspruch 16,
**dadurch gekennzeichnet,**
**dass** zumindest ein Griffteil (43) einen Einführschlitz (83) aufweist, durch den die Fixiereinrichtung (55) in das Griffteil (43) einführbar ist.

18. Vorrichtung nach einem der vorhergehenden Ansprüche,
**dadurch gekennzeichnet,**
**dass** das Basiselement (13) Abstützmittel (61) aufweist, die insbesondere auf gleicher Höhe wie Abstützmittel (37) des Verstellelements (15) angeordnet sind.
